# EUROPEAN PATENT APPLICATION

(11) **EP 1 430 892 A1**
(43) Date of publication of application: **23.06.2004**
(21) Application number: 02770213.3
(22) Date of filing: 25.09.2002
(51) Int. Cl.: A61K 31/192, A61K 9/08, A61K 47/04, A61K 47/14, A61P 1/00, A61P 1/04

(54) **AQUEOUS ECABET SODIUM SOLUTION PREPARATION**

(30) Priority: 27.09.2001 JP 2001296689
(71) Applicant: TANABE SEIYAKU CO., LTD., Chuo-ku, Osaka-shi, Osaka 541-8505 (JP)
(72) Inventor: NARISAWA, Shinji, Takatsuki-shi, Osaka 569-1041 (JP); SUGAYA, Kayo, Itami-shi, Hyogo 664-0836 (JP); ITO, Takahiro, Nishinomiya-shi, Hyogo 662-0934 (JP)
(74) Representative: Hayes, Adrian Chetwynd
(86) International application number: PCT/JP2002/009847
(87) International publication number: WO 2003/028716

(57) **Abstract**

An aqueous ecabet sodium solution preparation which contains at a concentration of 1 w/v% or more (in conversion to ecabet sodium) of sulfodehydroabietic acid, or its salt·ion, wherein said solution is adjusted to pH in the range of 7-8.5 with at least one pH buffer selected from a salt of a polycarboxylic acid and a salt of a polyphoshoric acid, and an inorganic base. The preparation is stable, less irritant and is suitable for intestinal administration.

## Description

### TECHNICAL FIELD

The present invention relates to an aqueous ecabet sodium solution preparation, in more detail, a stable, less irritant and easily administrable aqueous solution preparation containing at a high concentration sulfodehydroabietic acid or salt·ion thereof, which is the form of ecabet sodium (sulfodehydroabietic acid mono sodium salt pentahydrate; chemical name (+)-(1R,4aS,10aR)-1,2,3,4,4a,9,10,10a-octahydro-1,4a-dimethyl-7-(1-methylethyl)-6-sulfo-1-phenanthrencarboxylic acid 6-sodium salt pentahydrate), which is known as an agent for prophylaxis or treatment of inflammatory bowel disease, in the aqueous solution.

### BACKGRAUND ART

Inflammatory bowel disease means intractable inflammatory syndrome on large and small intestines due to various causes. It includes ulcerative colitis, which belongs to non-specific diffuse inflammatory disease which erodes mainly large intestinal membrane and forms erosion and ulcer whose cause is unknown, and Crohon's disease, which belongs to non-specific granulomatous inflammatory disease with fibrification or ulcer whose cause is unknown. It further includes the syndrome on an intestinal tube due to Behchet's disease, which is a systemic inflammatory disease.

Each cause of ulcerative colitis, Crohon's disease, Behchet's disease is not cleared, but recently attention has come to be paid to the immunological mechanism thereof. An immunosuppressive agent, a steroid, salazosulfapyridine, 5-aminosalicylic acid, etc., are used for treatment of such inflammatory bowel disease. However, satisfactory effect by them is not observed depending on the patients, and there is much room for improvement in the side effect thereof. Therefore, it is desired that more effective and safer drugs are developed.

On the other hand, sulfodehydroabietic acid or its pharmaceutically acceptable salt represented by a following formula; 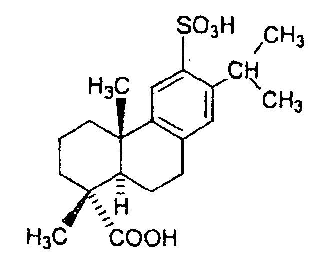 was developed, and this compound has been known to exhibit an inhibitory activity on acid secretion, pepsin secretion, etc., and to be useful as an agent for prophylaxis or treatment of peptic ulcer (gastric ulcer, duodenal ulcer), gastritis (Japanese Patent Publication A 58-77814, Japanese Patent Publication A 63-165361 and Japanese Patent Publication A 2-167258), dry eye, etc. (Japanese Patent Publication A 9-136832). Recently it was reported that this compound has an excellent activity for prophylaxis or treatment of inflammatory bowel disease, too (WO 01/34143).

However, a solution preparation containing at a concentration of 0.05-0.5w/v% sulfodehydroabietic acid mono sodium salt pentahydrate (ecabet sodium), whose pH is adjusted to 5-7 with sodium acetate or sodium dihydrogen phosphate, is used for prophylaxis or treatment of dry eye etc. And a suspension prepared by suspending the pulverized product of a solid preparation containing ecabet sodium in water is used for prophylaxis or treatment of inflammatory bowel disease.

### DISCLOSURE OF INVENTION

It is preferable to use an aqueous solution preparation in which ecabet sodium is dissolved at relatively high concentration for treatment of inflammatory bowel disease, but in order to lessen irritation on an inflammatory region, it is necessary to maintain pH of the aqueous solution near to pH of body fluid and to keep the osmotic pressure of the aqueous solution isotonic or less.

Furthermore, it has come to be known that since the solubility of ecabet sodium in an aqueous solution is low at a pH less than pH 7, and its solubility is greatly changeable depending on temperature, it is difficult to obtain a stable aqueous ecabet sodium solution preparation of high concentration without an appropriate pH control.

The present inventors have intensively studied to solve the above problem, namely to obtain a stable, less irritant and easily administrable aqueous solution preparation containing sulfodehydroabietic acid or its salt·ion, which is a form in the solution of ecabet sodium. The inventors have found that an aqueous ecabet solution preparation which contains at a rate of 1 w/v% or more (in conversion to ecabet sodium) of sulfodehydroabietic acid or its salt·ion, together with at least one buffer selected from a salt of a polycarboxylic acid and a salt of polyphosphoric acid, and an inorganic base, and whose pH is kept in the range of 7-8.5, is preferably used for treatment of inflammatory bowel disease. Thus the present invention has been completed.

### BRIEF DESCRIPTION OF DRAWINGS

Figure 1 shows a cross section of a flexible vessel for intestinal administration.
Figure 2 shows a cross section of another flexible vessel for intestinal administration.

### BEST MODE FOR CARRYING OUT THE INVENTION

In an aqueous solution preparation of the present invention, the concentration of sulfodehydroabietic acid, or its salt·ion, which is existing in a solution of ecabet sodium is high, but crystals of ecabet sodium do not precipitate from the solution by any change of temperature or occurrence of local concentration gradient without any special reservation.

Furthermore, the aqueous solution preparation of the present invention is not only adjusted to pH near to pH of body liquid, but also pH change is small during storage. The concentration of sulfodehydroabietic acid, or its salt· ion is high, but because increase of the osmotic pressure by pH adjustment and stabilization is controlled, and it is possible to control the solution isotonic or less, the aqueous solution preparation of the present invention shows a little stimulation or irritation at an inflammatory region in case of application.

The aqueous solution preparation of the present invention contains at a concentration of 1 w/v% or more (in conversion to ecabet sodium) of sulfodehydroabietic acid, or its salt·ion, and the solution is adjusted to a pH in the range of 7-8.5 with at least one pH buffer selected from a salt of a polycarboxylic acid and a salt of a polyphoshoric acid, and an inorganic base.

Sulfodehydroabietic acid or its salt is a known compound, and can be prepared for example, by the method described in Japanese Patent Publication A 58-77814, Japanese Patent Publication A 63-165361, Japanese Patent Publication A 2-167258, or in the similar manner thereto.

The concentration of sulfodehydroabietic acid, or its salt · ion in the solution preparation of the present invention is 1 w/v% or more, preferably 1-5 w/v%, more preferably 2-4 w/v% in conversion to ecabet sodium. In the aqueous solution preparation, water molecules included in ecabet sodium are incorporated into a solvent, namely water and further, the compound itself is in the status that mono sodium salt of sulfodehydroabietic acid, its monoanion and its dianion co-exist, and it does not exist in ecabet sodium. However the concentration in the solution is presented as the concentration of ecabet sodium.

The aqueous solution preparation of the present invention is not only limited to an aqueous solution prepared by dissolving ecabet sodium itself, but an aqueous solution having the same ingredients as the solution in which ecabet sodium is dissolved should be included in the aqueous solution preparation of the present invention, regardless of the kind of ingredients dissolved in an aqueous medium. For example, if a solution having the same ingredients as the ingredients of the present invention is obtained by dissolving free sulfodehydroabietic acid in a solution containing sodium ion, such a solution should be included in the aqueous solution preparation of the present invention, too.

A salt of a polycarboxylic acid used as a pH buffer of the aqueous solution preparation of the present invention includes a salt of an organic acid having plural carboxylic acid moieties in one molecule. Examples of such organic acids are a saturated or unsaturated dicarboxylic acid, e.g., malonic acid, succinic acid, malic acid, fumaric acid, maleic acid, tartaric acid, mesaconic acid, glutaric acid, and a tricarboxylic acid, e.g., citric acid. Salts of these polycarboxylic acids include alkali metal salts such as sodium salt and potassium salt. Preferable salts of polycarboxylic acids include sodium malate, sodium fumarate, sodium maleate, sodium tartrate and sodium citrate, most preferably trisodium citrate.

Salts of polyphosphoric acids used as a pH buffer in the aqueous solution preparation of the present invention mean a salt consisted of an anion of a linear condensed phosphoric acid represented by a formula: (PₙO₃ₙ₊₁) ⁽ⁿ⁺²⁾⁻ (wherein n is an integer of 2 or more) and a conjugated cation. An example of such salts includes a salt formed of an anion of diphosphoric acid, triphosphoric acid or tetraphosphoric acid and an alkali metal ion, such as sodium ion and potassium ion. Preferable salts of a polyphosphoric acid include an alkali metal salt with diphosphoric acid, triphosphoric acid and tetraphosphoric acid, especially preferably sodium triphosphate.

The concentration of these pH buffers is 0.01 to 2 w/v%, preferably 0.01 to 1.5 w/v%, but the concentration of a salt of a polycarboxylic acid is preferably 0.5 to 1.5 w/v% and the concentration of a salt of a polyphosphoric acid is preferably 0.01 to 0.5 w/v%.

Inorganic salts used in the aqueous solution preparation of the present invention include an alkali metal hydroxide, such as sodium hydroxide and potassium hydroxide, and an alkaline earth metal hydroxide, such as calcium hydroxide and magnesium hydroxide, preferably sodium hydroxide.

The inorganic base is added in the amount needed in order to adjust pH of the aqueous solution into 7 to 8.5, preferably 7.2 to 8.2. The addition is carried out by adding an aqueous inorganic base solution with stirring under monitoring pH of the aqueous solution by the use of a pH meter after addition or dissolution of other ingredients. In that case, the inorganic base itself or the concentrated aqueous solution thereof having a concentration of 0.2 to 10 w/v%, preferably 1 to 8 w/v%, more preferably 3 to 5 w/v% is preferably added in order to prevent the decrease of the concentration of sulfodehydroabietic acid, or its salt·ion.

The preparation method for the aqueous solution preparation of the present invention is not limited, but may be carried out by adding or dissolving under warming a determined amount of ecabet sodium to an aqueous medium, such as distilled water for injection and purified water, and adding a determined amount of a pH buffer and if desired, an additive(s) mentioned below to the mixture and then, adding an inorganic base to adjust pH in a range of 7 to 8.5 to prepare a stable solution.

An alternate method is a method that consists of adding or dissolving under warming simultaneously ecabet sodium, a pH buffer and other additives, and then adjusting pH of the solution by adding an inorganic base to prepare a stable solution.

To the aqueous solution preparation of the present invention may be, if necessary added a preservative, a viscosity-increasing agent, a solubilizing agent, an isotonic agent, and the like.

The preservative is added to prevent the growth of microorganisms during storage of the aqueous solution preparation, and a non ionic preservative is preferably used in order not to give a large effect to the osmotic pressure of the aqueous solution. For example, a lower alkyl ester of p-hydroxybenzoic acid (e.g., methyl ester, ethyl ester, propyl ester, or butyl ester of p-hydroxybenzoic acid), dehydroacetic acid, etc., preferably p-hydroxybenzoic acid methyl ester, ethyl ester and propyl ester, the small amount of which can suppress the growth of microorganisms, are used. The preservative may be, if necessary used in combination of two or more of them. The content of the preservative is usually in the range of 0.05 to 0.2 w/v%, preferably 0.07 to 0.15 w/v%.

The viscosity-increasing agent is added to the aqueous solution, so that the solution is easily retained around the damaged region when the aqueous solution preparation of the present invention is per rectally administered. The viscosity-increasing agent includes xanthan gum (Rhodigel prepared by Rhone-Poulenc, etc.), sodium carboxymethyl cellulose (Sodium carboxymethyl cellulose prepared by Daicel Chemical Ind., etc.).

The solubilizing agent includes polyethylene glycol (Polyethylene glycol prepared by Katayama Chemical, Inc. or Macrogol prepared by NOP Corp., etc.), a sugar fatty acid ester (Ryoto sugar ester prepared by Mitsubishi Chemical Corp. ltd., etc.), a polyoxyethylenepolyoxypropylene glycol (Pluronic prepared by Asahi Denka Co., Ltd., or Plonon prepared by NOP Corp.), a glycerin fatty acid ester (Nikkol MGS series prepared by Nikko Chemicals), a sorbitan fatty acid ester (Nikkol SL, SP, SS, SO, SI series prepared by Nikko Chemicals, or Nonion prepared by NOF Corp.), an alkanolamine (diethanolamine, triethanolamine, isopropanolamine, etc.), and an alcohol (ethanol, propyleneglycol, etc.).

The isotonic agent includes a water soluble inorganic salt such as sodium chloride, a sugar such as D-mannitol, and may be used if necessary.

The ecabet sodium aqueous solution preparation of the present invention is useful especially for prophylaxis and treatment for inflammatory bowel disease.

The inflammatory bowel disease is not limited to a narrow sense thereof, such as Chron's disease and ulcerative colitis, but means a broad sense including intestinal syndrome in Bechet's disease, hemorrhagic rectal ulcer, pouchtis, intestinal tuberculosis, ischemic enteritis, drug-induced colitis, radiation induced enteritis, and infectious enteritis.

The terms "prophilaxis and treatment" include the improvement of symptoms, the prevention of exacerbation, the maintenance of remission, the prevention of recrudescence, the prevention of constriction of digestive tube, the prevention of recrudescence after the surgical operation, etc.

The aqueous solution preparation of the present invention is preferably intestinally administered between rectal and sigmoid, or directly administered into intestinal tube via an artificial anus.

The dose may vary according to the administration route, age, body weight and condition of a patient, or severity of the disease to be cured, but the daily dose thereof for an adult is usually in the range of about 10 to 300 mg/kg/body weight, preferably in the range of about 20 to 150 mg/kg/body weight in conversion to ecabet sodium.

Furthermore, the sterilization of the aqueous solution preparation of the present invention is carried out by, if necessary, filtering with a membrane before filling into a vessel or, if necessary, heating a vessel itself after filling into a vessel.

The aqueous solution preparation of the present invention may be filled in a hard vessel, such as a grass bottle and a plastic bottle, and if necessary, the aqueous solution preparation may be intestinally administered with a device for intestinal administration (an enemator). But the solution preparation may be filled in a flexible vessel suitable for intestinal administration and the aqueous solution preparation can be directly administered from this vessel.

The flexible vessel suitable for intestinal administration includes various forms of vessels which consist of a main vessel portion (sphere, oval, bellows, etc.) in which the aqueous solution preparation is filled and a nozzle portion for injection (which may have a function to prevent reverse flow). Materials for a flexible vessel suitable for intestinal injection include a thermoplastic resin which is thermo resistant to some extent (e.g., polyolefine resin, such as low density polyethylene, high density polyethylene, polypropylene, polymethylbutene and polymethylpentene; vinyl acetate resin, such as ethylene vinylacetate copolymer and polyvinylacetate).

Furthermore, after filling the flexible vessel suitable for intestinal administration may be shielded from light with a film prepared by laminating an aluminum middle layer with a polymer sheet, such as polyethylene, polyoropyrene, nylon, ethylenevinylalcohol copolymer, polystyrene, cellophane, etc., a film prepared by depositing aluminum on a polymer sheet, such as polyethylene, polypropylene, nylon, ethylenevinylalcohol copolymer, etc., or a film prepared by incorporating a shielding substance into polyethylene, polypropylene, nylon, ethylenevinylalcohol copolymer, etc.

The aqueous solution preparation of the present invention and its efficacy will be illustrated in more detail by the following Examples and Experiments, but the present invention should not be limited by them.

### Example 1

Ecabet sodium (4g), methyl p-hydroxybenzoate (0.1g) and propyl p-hydroxybenzoate (0.02g) were dissolved under warming in purified water (80ml). After trisodium citrate (1g) was added under stirring to the solution, the solution was adjusted to pH 7.9 with an aqueous sodium hydroxide solution and then, additional purified water was added thereto to give an aqueous ecabet sodium solution preparation (100ml).

### Example 2

Ecabet sodium (4g), methyl p-hydroxybenzoate (0.1g) and propyl p-hydroxybenzoate (0.02g) were dissolved under warming in purified water (80ml). After sodium triphosphate (0.03g, chemical formula: Na₅P₃O₁₀) was added under stirring to the solution, the solution was adjusted to pH 7.9 with an aqueous sodium hydroxide solution and then, additional purified water was added thereto to give an aqueous ecabet sodium solution preparation (100ml).

### Example 3

Methyl p-hydroxybenzoate (0.1g) and propyl p-hydroxybenzoate (0.02g) were dissolved in ethanol (1g). On the other hand ecabet sodium (2g) and sodium triphosphate (0.2g) were added to purified water (80ml). After the ethanol solution was added under stirring to the mixture, the mixture was adjusted to pH 7.4 with an aqueous sodium hydroxide solution and then, additional purified water was added thereto to give an aqueous ecabet sodium solution preparation (100ml).

### Example 4

Ecabet sodium (2g), methyl p-hydroxybenzoate (0.1g), propyl p-hydroxybenzoate (0.02g) and trisodium citrate (1g) were dissolved under warming in purified water (80ml). After the solution was adjusted to pH 7.4 with an aqueous sodium hydroxide solution and then, additional purified water was added thereto to give an aqueous ecabet sodium solution preparation (100ml).

### Example 5

Ecabet sodium (2g), methyl p-hydroxybenzoate (0.1g) and trisodium citrate (1g) were dissolved under warming in purified water (80ml). After the solution was adjusted to pH 7.4 with an aqueous sodium hydroxide solution and then, additional purified water was added thereto to give an aqueous ecabet sodium solution preparation (100ml).

### Example 6

Methyl P-hydroxybenzoate (0.1g), xanthan gum (0.175g, Rhodigel 200, average molecular weight: 2,000,000 prepared by Rhone-Poulenc) were dissolved in polyethylene glycol (1.5g, Polyethylene glycol 400, average molecular weight: 400 prepared by Katayama Chemical, Inc.). On the other hand, ecabet sodium (4g) and sodium triphosphate (0.2g) were added to purified water (80ml). After the polyethylene glycol solution was added under stirring to the mixture, the mixture was adjusted to pH 8 with an aqueous sodium hydroxide solution and then, purified water was added thereto to give an aqueous ecabet sodium solution preparation (100ml).

### Example 7

Ecabet sodium (4g), polyethylene glycol (8g, Polyethylene glycol 4000, average molecular weight: 4000 prepared by Katayama Chemical, Ind.), methyl p-hydroxybenzoate (0.1g), and trisodium citrate (1g) were dissolved under warming in purified water (80ml). After the solution was adjusted to pH 8 with an aqueous sodium hydroxide solution and then, purified water was added thereto to give an aqueous ecabet sodium solution preparation (100ml).

### Example 8

Butyl p-hydroxybenzoate (0.005g) and sodium carboxymethyl cellulose (1.4g, Sodium carboxymethyl cellulose 1260, mean degree of etherification: 0.91 prepared by Daicel Chemical Ind.) were dissolved in polyethylene glycol (1.5g, Polyethylene glycol 400, average molecular weight: 400 prepared by Katayama Chemical, Inc.). On the other hand, ecabet sodium (4g) and sodium triphosphate (0.2g) were added to purified water (80ml). After the polyethylene glycol solution is added to the mixture, the mixture was adjusted to pH 8 with an aqueous sodium hydroxide solution and then, purified water was added thereto to give an aqueous ecabet sodium aqueous solution preparation (100ml).

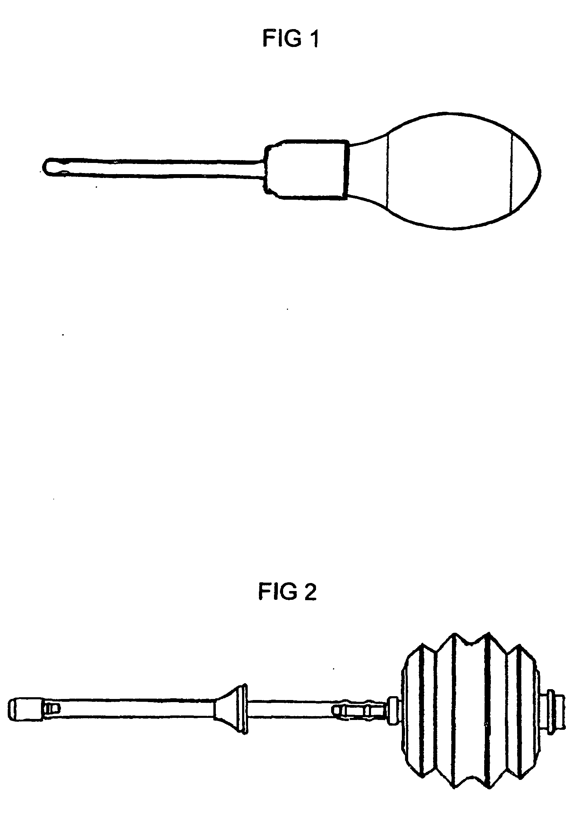

Each of the aqueous ecabet sodium solution preparations prepared by Examples 1 to 8 filled in a flexible vessel such as shown in figure 1 or 2 can be used.

### Comparative example

After ecabet sodium (4g) was added to purified water (80ml), the solution was adjusted to pH 8 with an aqueous sodium hydroxide solution. Purified water was further added to the solution to give an aqueous ecabet solution preparation (100ml).

### Experiment 1 (Stability of solution)

Two sets of each solution of Examples 1, and 2, and Comparative example were filled in a grass bottle. One set thereof was stored at 60°C for a week and the other set was stored at 5°C for a month.

pH on each solution was measured with a pH meter (Horiba F-22 manufactured by Horiba, Ltd.). The value obtained by subtracting pH value on the solution stored at 5°C for a month from pH value on the solution stored at 60°C for a week is presented as ΔpH. The result is shown in Table 1.

**Table 1**

| | Example 1 | Example 2 | Comparative example |
|---|---|---|---|
| Buffer (concentration) (concentration) | Trisodium citrate (1g/100ml) | Sodium triphosphate (0.03g/100ml) | - |
| pH after being stored at 5°C for a month | 7.90 | 7.96 | 8.15 |
| ΔpH after being stored at 60°C for a week | 0.05 | 0.05 | 0.52 |

As is clear from the above result, when trisodium citrate and sodium triphosphate are used as a buffer, the concentration of proton decreases only by 11% (ΔpH=0.05) even at storage at 60°C for a week. But when any buffer is not used, of the concentration of proton decreases by 70% (ΔpH=0.52) at the same conditions and the pH was beyond the preferable range for a preparation for intestinal application.

### Experiment 2

Each solution of Examples 4 and 5 (each 100ml) was filled in a grass bottle. Separately, a bacteria suspension containing Pseudomonas aeruginosa (ATCC 9027) at a rate of 10⁷ to 10⁸cells/ml was prepared. The bacteria suspension (1ml) was inoculated in each solution prepared above and the resulting mixture was maintained at 25°C in a thermostat.

After 1 week, a sample was taken from each of the bottle and the number of living cells was counted according to agar plate mixed dilution method, Test method containing micro organisms (Japanese Pharmacopoeia 14th ed., page 72-7θ). The result was shown in Table 2.

**Table 2**

| | Example 4 | Example 5 |
|---|---|---|
| Additive (amount) (amount) | Methyl p-hydroxybenzoate (0.1g/100ml) and Propyl p-hydroxybenzoate (0.02g/100ml) | Methyl zoate p-hydroxybenzoate (0.1g/100ml) |
| Inoculated amount of bacteria suspension | 1ml | 1ml |
| Number of living cell after 1 week | none/ml | 49 cells/ml |

When a usual preservative (benzoic acid) was added to the aqueous ecabet sodium solution and then to the solution were inoculated bacteria (Staphylococcus aureus ATCC 6538, Escherichia coli ATCC 8739, Pseudomonas aeruginosa ATCC 9.027), and fungi (Candida albicans ATCC 10231, Aspergillus niger ATCC 16404) and the mixture was maintained at 25°C in a thermostat, the number of living cells of Pseudomonas aeruginosa was the largest. Even though said bacterium was used as a standard bacterium, the growth of said bacteria can be effectively inhibited by adding methyl p-hydroxybenzoate or a combination thereof with propyl p-hydroxybenzoate to the aqueous ecabet sodium solution as is shown in the above result.

### INDUSTRIAL APPLICABILITY

The aqueous ecabet sodium solution preparation of the present invention which is added with a specific pH buffer and adjusted its pH to 7-8.5 with an inorganic base can be stored for the long term in spite of the high concentration of sulfodehydroabietic acid or its salt-ion and exhibits an effect for prophylaxis or treatment for inflammatory bowel disease.

## Claims

1. An aqueous ecabet sodium solution preparation which contains 1 w/v% or more (in conversion to ecabet sodium) of sulfodehydroabietic acid, or its salt · ion, wherein said solution is adjusted to pH in the range of 7-8.5 with at least one pH buffer selected from a salt of a polycarboxylic acid and a salt of a polyphoshoric acid, and an inorganic base.

2. The preparation of claim 1, wherein the pH buffer is at least one selected from an alkali metal salt of a polycarboxylic acid and an alkali metal salt of a polyphoshoric acid, and the inorganic base is at least one selected from an alkali metal hydroxide and an alkaline earth metal hydroxide.

3. The preparation of claim 2, wherein the pH buffer is at least one selected from an alkali metal salt of a dicarboxylic acid and a tricarboxylic acid and an alkali metal salt of diphoshoric acid, triphosphoric acid and tetraphosphoric acid, and the inorganic base is an alkali metal hydroxide.

4. The preparation of claim 3, wherein the pH buffer is trisodium citrate or sodium triphosphate, and the inorganic base is sodium hydroxide.

5. The preparation of claim 3, wherein the pH buffer is sodium triphosphate, and the inorganic base is sodium hydroxide.

6. The preparation of claim 3, wherein the pH buffer is trisodium citrate, and the inorganic base is sodium hydroxide.

7. The preparation of any one of claims 1 to 6, wherein the concentration of the pH buffer is 0.01 to 2 w/v%.

8. The preparation of claim 5, wherein the concentration of sodium triphosphate is 0.01 to 0.5 w/v%.

9. The preparation of claim 6, wherein the concentration of trisodium citrate is 0.5 to 1.5 w/v%.

10. The preparation of any one of claims 1 to 9, wherein the concentration (in conversion to ecabet sodium) of sulfodehydroabietic acid, or its salt·ion is 1 to 5 w/v%.

11. The preparation of claim 10, wherein the concentration (in conversion to ecabet sodium) of sulfodehydroabietic acid, or its salt·ion is 2 to 4 w/v%.

12. The preparation of claim 1 which contains further at least one compound selected from lower alkyl esters of p-hydroxybenzoic acid.

13. The preparation of claim 12, wherein the ester of p-hydroxybenzoate is methyl p-hydroxybenzoate, ethyl p-hydroxybenzoate or propyl p-hydroxybenzoate.

14. The preparation of claim 12 or 13, wherein the concentration of an ester of p-hydroxybenzoic acid is 0.05 to 0.2 w/v%.

15. The preparation of claim 12 or 13, wherein the concentration of an ester of p-hydroxybenzoic acid is 0.07 to 0.15 w/v%.

16. The preparation of any one of claims 1 to 15 for treatment of inflammatory bowel disease.

17. The preparation of claim 16 for intestinal administration.

18. The preparation of claim 16 which is filled in a flexible vessel for intestinal administration.
